(19) **Europäisches Patentamt**
**European Patent Office**
**Office européen des brevets**

(11) **EP 4 169 541 A1**

(12) **EUROPEAN PATENT APPLICATION**

(43) Date of publication:
26.04.2023 Bulletin 2023/17

(21) Application number: 22199080.7

(22) Date of filing: 08.11.2011

(51) International Patent Classification (IPC):
**A61L 27/26** (2006.01) **A61L 27/50** (2006.01)
**A61L 27/52** (2006.01)

(52) Cooperative Patent Classification (CPC):
(C-Sets available)
**A61L 27/26; A61L 27/50; A61L 27/52;**
A61L 2300/402; A61L 2400/06 (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 08.11.2010 PCT/IB2010/002846
23.11.2010 PCT/IB2010/003008

(62) Document number(s) of the earlier application(s) in
accordance with Art. 76 EPC:
**21150280.2 / 3 818 993**
**16193473.2 / 3 138 586**
**11799644.7 / 2 637 710**

(71) Applicant: **Allergan Industrie, SAS**
**74370 Pringy (FR)**

(72) Inventors:
• **Lebreton, Pierre**
**74000 Annecy (FR)**
• **Guetta, Olivier**
**74000 Annecy (FR)**

(74) Representative: **Hoffmann Eitle**
**Patent- und Rechtsanwälte PartmbB**
**Arabellastraße 30**
**81925 München (DE)**

Remarks:
•This application was filed on 30.09.2022 as a
divisional application to the application mentioned
under INID code 62.
•Claims filed after the date of receipt of the divisional
application (Rule 68(4) EPC).

(54) **SOFT TISSUE FILLER**

(57) The present invention provides a soft tissue filler composition comprising (1) a crosslinked hyaluronic acid (HA) component comprising a crosslinked mixture of (i) a first, low molecular weight HA material having a weight average molecular weight of between 0.20 MDa and 0.99 MDa, and (ii) a second, high molecular weight HA material having a weight average molecular weight of between 1.0 MDa and 4.0 MDa, wherein the weight average molecular weight of the second, high molecular weight HA material is at least twice that of the first, low molecular weight HA material, wherein the HA materials are hyaluronic acid or one or more salts thereof, wherein the crosslinked HA component has a HA concentration of between 15.0 mg/g and 20.0 mg/g, and wherein the crosslinking is achieved by use of a crosslinking agent; and (2) an uncrosslinked HA component which is HA or a salt thereof, and which has a weight average molecular weight of at least 2.0 MDa and is present in the composition in an amount of less than 5.0% w/w.

Processed by Luminess, 75001 PARIS (FR)

**(Cont. next page)**

(52) Cooperative Patent Classification (CPC): (Cont.)

C-Sets
**A61L 27/26, C08L 5/08**

**Description**

The present invention generally relates to injectable soft tissue fillers and more specifically relates to hyaluronic acid-based dermal and subdermal fillers having improved properties.

Skin is composed of the epidermis and the dermis. Below these layers lies the hypodermis, which is not usually classified as a layer of skin. The hypodermis is also commonly referred to as subcutaneous fat layer, or subcutaneous tissue. The outermost epidermis is made up of stratified squamous epithelium with an underlying basement membrane. It contains no blood vessels, and is nourished by diffusion from the dermis. The main type of cells which make up the epidermis are keratinocytes, with melanocytes and langerhans cells also present. This layer of skin is responsible for keeping water in the body and keeping other harmful chemicals and pathogens out.

The dermis lies below the epidermis and contains a number of structures including blood vessels, nerves, hair follicles, smooth muscle, glands and lymphatic tissue. The dermis (or corium) is typically 3-5 mm thick and is the major component of human skin. It is composed of a network of connective tissue, predominantly collagen fibrils providing support and elastic tissue providing flexibility. The main cell types are fibroblasts, adipocytes (fat storage) and macrophages. The hypodermis lies below the dermis. Its purpose is to attach the skin to underlying bone and muscle as well as supplying it with blood vessels and nerves. It is made up of loose connective tissue and elastin. The main cell types are fibroblasts, macrophages and adipocytes. The hypodermis contains 50% of body fat. Fat serves as padding and insulation for the body. Hyaluronic acid (HA) is a part of the dermis composition and is a major component of the extra cellular matrix.

Facial aging occurs as the result of several factors: inherent changes within the skin, effects of gravity, facial muscles acting on the skin (dynamic lines), soft tissue loss or shift and bone loss and loss of tissue elasticity. The skin ages when the epidermis begins to thin, causing the junction with the dermis to flatten. Collagen decreases as a person ages and the bundles of collagen, which gives the skin turgor, become looser and lose strength. When the skin loses elasticity, it is less able to resist stretching. Coupled with gravity, muscle pull and tissue changes, the skin begin to wrinkle. Water loss and breakdown of bonds between cells also reduces the barrier function of the skin, which can cause the skin's pore size to increase.

As a person ages, the face loses volume, soft tissue, and fat. The appearance of jowls and folds are usually caused by the drooping of facial tissues and folding of areas where the muscles below are attached to the skin. As part of the reduction in soft tissue, the aging face appears more hollow.

More specifically, in various facial areas, such as forehead, eyes, nose, midface and lower face, changes relating to aging have been well documented. In forehead area, the forehead and brow droop over time, which lowers the eyebrows and causes the upper eyelid skin to bunch. Forehead lines appear when one tries to hold the brows and eyelids up to counteract these changes. It is well known that the eyes are often the first facial feature to show signs of aging. Skin changes around the eyes occur earlier than in the rest of the face since the skin is thinner around the eyes. The skin here contains fewer glands and is subjected to constant blinking, squinting, rubbing, and pulling. The midface ages when the cheeks begin to droop, causing nasolabial folds. Nasolabial folds are the lines that run from the sides of the nose to the corners of the mouth. These folds have been treated with facial fillers. In the nose area, as a person ages, the nose elongates. Common causes of elongation are thinning of the soft tissue and loss of elasticity, which causes "drooping of the tip" and unmasking of the bone, creating a new hump. In the lower face area, as the face ages, facial tissues descend. This results in the so-called "laugh lines".

Folds and lines in this area have been treated with facial fillers. Further down on the face, the corners of the mouth may droop and descent of the jowls can create folds often referred to as "marionette" lines. Furthermore, jowls form when the cheeks sag around a fixed point along the jaw where the facial muscles attach to the jawbone. The facial muscles continue down into the neck as a sheet called the platysma muscle. This muscle often gaps in the center of the neck, creating two bands.

Various injectables have been used for restoring tissue loss in the face. In the past, injectable collagen has been used as a soft-tissue filler to fill wrinkles, lines and scars on the face. Collagen is a naturally occurring protein that supports various parts of the body including skin, tendons and ligaments. Fat injections have been used for years to add volume, fill wrinkles, lines and enhance the lips. Fat injections involve taking fat from one part of the patient's body (abdomen, thighs or buttocks) and injecting it beneath the facial skin.

Hyaluronic acid (HA), now one of the most commonly used components of cosmetic dermal fillers, is introduced into aging skin to add volume and minimize wrinkles and lines. Hyaluronic acid is a naturally occurring, water soluble polysaccharide, specifically a glycosaminoglycan, which is a major component of the extra-cellular matrix and is widely distributed in animal tissues. The identical structure of hyaluronic acid in all species and tissues makes this polysaccharide an ideal substance for use as a bio-material in health and medicine. Hyaluronic acid is present in many places in the human body. It gives volume to the skin, shape to the eyes and elasticity to the joints.

Different from animal derived hyaluronic acid, non-animal derived hyaluronic acid is free from animal proteins. This limits the risk of animal based disease transmissions or development of allergic reactions to animal proteins.

HA, also known as hyaluronan, has excellent biocompatibility and, unlike collagen, does not require any skin testing before implantation. In addition, HA has the ability to bind to large amounts of water, making it an excellent volumizer of soft tissues.

The development of HA-based fillers which exhibit ideal *in vivo* properties as well as ideal surgical usability has proven difficult. For example, HA-based fillers that exhibit desirable stability properties *in vivo,* can be so highly viscous that injection through fine gauge needles is difficult. Conversely, HA-based fillers that are relatively easily injected through fine gauge needles often have relatively inferior stability properties *in vivo.*

One method to overcome this problem is to have an adequate design of crosslinked HA-based fillers. Crosslinked HA is formed by reacting uncrosslinked HA with a crosslinking agent under suitable reaction conditions.

It is generally accepted that HA-based dermal fillers having a high viscosity, for example, those that are highly crosslinked and/or made of high molecular weight HA and/or having a high HA concentration tend to last longer in the body. Conversely, it is generally accepted that HA-based dermal fillers having a low viscosity, for example, those that are more lightly crosslinked and/or made up of low molecular weight HA and/or have a low HA concentration, may have a shorter duration in the body. Naturally, injection of a high viscosity material through a needle is relatively more difficult, and generally requires a smaller gauge needle (for instance, 21G, 23G vs 27G, 30G) than injection of a relatively low viscosity material. It has proven difficult to develop an HA based composition that is both easy to inject through a high gauge needle (i.e. thin needle) and which has extended duration in the body. Surprisingly, many of the long lasting, highly injectable compositions of the present invention include a high percentage of relatively low molecular weight HA at relatively low concentrations.

The present invention addresses these and other issues by providing an injectable HA based dermal filler that has enhanced longevity and is extrudable through a fine needle, thus being more comfortable for the patient during injection and requiring fewer repeated visits to the physician.

**Summary**

The present invention relates to soft tissue fillers, for example, dermal and subdermal fillers, based on hyaluronic acid (HA) and pharmaceutically acceptable salts of HA, for example, sodium hyaluronate (NaHA). In some embodiments of the invention, HA-based compositions are provided which include a therapeutically effective amount of at least one anesthetic agent, for example, but not limited to, lidocaine.

The present HA-based compositions are relatively highly viscous at rest but with low viscosity under high shear rate, thus facilitating injection even through a fine (i.e. high gauge, e.g. 27G, 30G, 31G, 33G) needle, and have enhanced longevity in the body. The compositions may last up to four months, six months, 12 months or longer, depending on various factors such as where the compositions are introduced in the body. Methods for preparing such HA-based compositions are also provided as well as products made by such methods.

In a broad aspect of the invention, a soft tissue filler composition is provided which generally comprises a crosslinked hyaluronic acid (HA) component and an uncrosslinked hyaluronic acid component. The crosslinked HA component itself comprises a mixture of a first, low molecular weight HA material and a second, higher molecular weight HA material. The first molecular weight HA material has a relatively low molecular weight and the second molecular weight HA material has a relatively high molecular weight.

Generally, the crosslinked HA component includes more than 50%, for example, at least 70%, for example, about 90% by weight of the first, low molecular weight HA.

The uncrosslinked HA component is a relatively high molecular weight HA material, for example, a HA having a molecular weight of at least about 1.0MDa, and may be present in the composition in an amount of less than about 10%, for example, less than 5.0%, for example, less than about 2.0%, for example, less than about 1.0%, for example, about 0.95% w/w.

The crosslinking agent may be any suitable crosslinking agent, but in a particular embodiment, the crosslinking agent is selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether.

In some embodiments, the compositions further comprise at least active agent, for example, an anesthetic agent combined with said crosslinked HA component.

The crosslinked HA component has a total HA concentration of at least 10.0 mg/g. In certain specific embodiments, the crosslinked HA component has a total HA concentration of at least about 10.0 mg/g, for example, about 15.0 mg/g, about 17.0 mg/g, about 17.5 mg/g or about 20.0 mg/g, or about 25.0 mg/g.

Methods of making soft tissue filler compositions are also provided. In one embodiment, a method of making a soft tissue filler composition generally comprise the steps of providing a crosslinked hyaluronic acid (HA) gel comprising a mixture of a first molecular weight HA material and a second, different, for example, higher, molecular weight HA material, preparing a separate solution of an uncrosslinked hyaluronic acid of a relatively high molecular weight and combining the crosslinked gel with a small amount of the uncrosslinked solution. The compositions may include at least one anesthetic agent combined with said crosslinked HA component. The crosslinked HA component has a total HA concentration of at least 10 mg/g.

In embodiments of the invention including an anesthetic agent, the agent may comprise lidocaine. In a further embodiment, the amount of the anesthetic agent is present at a concentration between about 0.1% and about 5.0% by weight of the composition. In still another embodiment, the anesthetic agent is present at a concentration between about 0.2% and about 1.0% by weight of the composition. In one embodiment, the anesthetic agent is lidocaine and is present at a concentration of about 0.3% by weight of the composition.

In yet another embodiment, the composition has a complex viscosity of between about 50 Pa*s and about 450 Pa*s, for example, when measured at about 5 Hz with a rheometer using a cone/plate geometry (4cm/2°) at 25°C.

In one embodiment, the HA component is a gel, for example, a cohesive, hydrated gel. In one embodiment, the HA component is a crosslinked HA gel having no greater than about 1% to about 10% uncrosslinked HA.

In yet other embodiments, the HA component comprises greater than about 10%, for example, greater than about 15%, for example, up to or greater than about 20% uncrosslinked HA.

Further described herein is a soft tissue filler composition comprising: a HA component crosslinked with 1,4-butanediol diglycidyl ether (BDDE), said HA component having a degree of crosslinking of less than about 5%, for example, about 2%, and an anesthetic component having a concentration between about 0.1% and about 5.0% by weight of the soft tissue filler composition, wherein the anesthetic is lidocaine.

Further described herein are methods of preparing soft tissue filler compositions, the methods comprising the steps of: providing a HA component crosslinked with at least one crosslinking agent selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether or combinations thereof; adjusting the pH of said HA component to an adjusted pH above about 7.2; and adding a solution containing at least one anesthetic agent to the HA component having the adjusted pH to obtain a HA-based filler composition.

In another embodiment, the composition is sterilized, for example, by autoclaving, to form a sterilized composition and wherein the sterilized composition is stable at ambient temperature for at least about 12 months, for example, at least 18 months, at least about 24 months or more.

In still another embodiment, the adjusted pH is above about 7.5. In another embodiment, the method further comprises the step of homogenizing the HA component during or after the step of adding the solution containing the at least one anesthetic agent. In a further embodiment, the step of homogenizing comprises subjecting the composition to mixing with a controlled shear.

In another embodiment, the step of providing a HA component comprises providing dry NaHA material and hydrating the dry NaHA material in an alkaline solution to obtain an alkaline, uncrosslinked NaHA gel. In yet another embodiment, the alkaline NaHA gel has a pH greater than about 8.0. In still another embodiment the pH is greater than about 10.

In a further embodiment, the HA component comprises less than about 20% uncrosslinked HA and the crosslinked portion of the HA component has a degree of crosslinking of less than about 6% or less than about 5%.

Further described herein is a soft tissue filler composition comprising: a crosslinked hyaluronic acid (HA) component comprising up to 90% w/w of low molecular weight HA and no more than about 10% w/w of high molecular weight HA crosslinked with 1,4-butanediol diglycidyl ether (BDDE), said crosslinked HA component having a degree of crosslinking of less than about 5%, an anesthetic component such as lidocaine having a concentration between about 0.1% and about 5.0% by weight, and less than 5.0% w/w of an uncrosslinked component of high molecular weight HA.

The present invention further provides methods of preparing a soft tissue filler composition. In some embodiments, a method is provided comprising the steps of: providing dry NaHA material and hydrating the dry NaHA material in an alkaline solution to obtain an alkaline, uncrosslinked NaHA gel; crosslinking the NaHA gel with BDDE to form a crosslinked alkaline HA composition with a degree of crosslinking less than about 5% and a pH above about 7.2; adding a solution containing 0.3% lidocaine HCl to the HA component having the adjusted pH to obtain said HA-based filler composition; homogenizing the HA-based filler composition thereby forming a homogenized HA-based filler composition; adding an amount of a high molecular weight HA uncrosslinked gel to the composition, and sterilizing the homogenized HA-based filler composition thereby forming a sterilized HA-based filler composition.

## Definitions

Certain terms as used in the specification are intended to refer to the following definitions, as detailed below. Where the definition of terms departs from the commonly used meaning of the term, applicant intends to utilize the definitions provided below, unless specifically indicated.

Autoclave stable or stable to autoclaving as used herein describes a product or composition that is resistant to degradation such that the product or composition maintains at least one, and preferably all, of the following aspects after effective autoclave sterilization: transparent appearance, pH, extrusion force and/or rheological characteristics, hyaluronic acid (HA) concentration, sterility, osmolarity, and lidocaine concentration.

The term "about" in the context of numerical values will be readily understood by a person skilled in the art, and preferably means that specific values may be modified by +/- 10%. As regards endpoints of ranges, the modifier "about" preferably means that the lower endpoint may be reduced by 10% and the upper endpoint increased by 10%. It is also contemplated that each numerical value or range disclosed in this application can be absolute, i.e. that the modifier "about" can be deleted.

All numbers herein expressing "molecular weight" of HA are to be understood as indicating the weight average molecular weight (Mw) in Daltons.

The molecular weight of HA is calculated from an intrinsic viscosity measurement using the following Mark Houwink relation:

$$\text{Intrinsic Viscosity (m}^3\text{/kg)} = 9.78 \times 10^{-5} \times Mw^{0.690}$$

The intrinsic viscosity is measured according to the procedure defined European Pharmacopoeia (HA monograph N°1472, 01/2009).

High molecular weight HA as used herein describes a HA material having a molecular weight of at least about 1.0 million Daltons (mw $\geq 10^6$ Da or 1 MDa) to about 4.0 MDa. For example, the high molecular weight HA in the present compositions may have a molecular weight in the range about 1.5 MDa to about 3.0 MDa, or the high molecular weight HA may have a weight average molecular weight of about 2.0 MDa. In another example, the high molecular weight HA may have a molecular weight of about 3.0 MDa.

Low molecular weight HA as used herein describes a HA material having a molecular weight of less than about 1.0 MDa. Low molecular weight HA can have a molecular weight of between about 200,000 Da (0.2 MDa) to less than 1.0 MDa, for example, between about 300,000 Da (0.3 M Da) to about 750,000 Da. (0.75 MDa), up to but not exceeding 0.99 MDa.

Preferably, there is no overlap between the molecular weight distribution of the low and high molecular weight HA materials.

Preferably, the mixture of the low molecular weight HA and high molecular weight HA has a bimodal molecular weight distribution. The mixture may also have a multi-modal distribution.

In one aspect of the invention, the compositions comprise HA having a high molecular weight component and a low molecular weight component, and the high molecular weight component has a weight average molecular weight at least twice the weight average molecular weight of the low molecular weight component.

For example, the molecular weight ratio of the high molecular weight HA to the low molecular weight HA in the composition is at least 2:1.

For example, a composition in accordance with this aspect of the invention may include a low molecular weight component having a weight average molecular weight of about 500,000 Da, and a high molecular weight component having a weight average molecular weight of about, or at least about, 1.0 MDa.

In another example, a composition in accordance with the invention may include a low molecular weight component having a weight average molecular weight of about 800,000 Da, and a high molecular weight component having a weight average molecular weight of about, or at least about, 1.6 MDa.

In a similar aspect of the invention, methods for making a HA based composition are provided which generally include the steps of selecting a low molecular weight HA material and selecting a high molecular weight HA material having a molecular weight at least twice as high as the molecular weight of the low molecular weight material, combining these high and low molecular weight materials, and crosslinking these combined materials with a suitable crosslinking agent.

Degree of crosslinking as used herein refers to the intermolecular junctions joining the individual HA polymer molecules, or monomer chains, into a permanent structure, or as disclosed herein the soft tissue filler composition. Moreover, degree of crosslinking for purposes of the present disclosure is further defined as the percent weight ratio of the crosslinking agent to HA-monomeric units within the crosslinked portion of the HA based composition. It is measured by the weight ratio of crosslinker to HA monomers (crosslinker : HA monomers).

Uncrosslinked HA as used herein refers to individual HA polymer molecules that are not crosslinked, or are very lightly crosslinked (very low degree of crosslinking). Uncrosslinked HA generally remains water soluble.

Cohesive as used herein is the ability of a HA-based composition to retain its shape and resist compression. In some embodiments of the invention, cohesiveness includes the ability of the gel to absorb at least one time its weight of water without breaking into small pieces of gel. Cohesiveness is affected by, among other factors, the molecular weight ratio of the initial uncrosslinked HA, the degree of crosslinking, the amount of residual uncrosslinked HA following crosslinking or added to the composition.

**Detailed Description**

The present disclosure generally relates to soft tissue fillers, for example, injectable dermal and subdermal fillers, based on hyaluronic acids (HA) and pharmaceutically acceptable salts of HA, for example, sodium hyaluronate (NaHA). In one aspect, HA-based compositions described herein include a therapeutically effective amount of at least one anesthetic agent, for example, lidocaine. The present HA-based compositions may include at least one anesthetic agent, for example, lidocaine. Methods of making these compositions are also provided.

In one aspect of the invention, the compositions maintain at least one of, or all of, the following aspects after effective autoclave sterilization and/or prolonged storage: transparent appearance, pH for use in a patient, extrusion force and/or rheological characteristics, HA concentration, sterility, osmolarity, and lidocaine concentration. Methods or processes of preparing such HA-based compositions are also provided as well as products made by such methods or processes.

As used herein, hyaluronic acid (HA) can refer to any of its hyaluronate salts, and includes, but is not limited to, sodium hyaluronate (NaHA), potassium hyaluronate, magnesium hyaluronate, calcium hyaluronate, and combinations thereof. Both HA and pharmaceutically acceptable salts thereof can be used in this invention.

Generally, the concentration of HA in the compositions described herein is preferably at least 10 mg/mL and up to about 40 mg/mL.

In certain embodiments, the HA concentration in the crosslinked component of the present compositions is no greater than about 25 mg/g, for example, about 20.0 mg/g, for example, about 18.0 mg/g, about 17.0 mg/g, about 16.0 mg/g, about 15.0 mg/g, about 13.0 mg/g, about 12.0 mg/g, about 11.0 mg/g or about 10.0 mg/g. In one embodiment, the HA concentration in the crosslinked component is about 17.5 mg/g. In another embodiment the HA concentration in the crosslinked component is 15.0mg/g. In another embodiment, the HA concentration in the crosslinked component is about 25.5 mg/g.

In addition, in embodiments with anesthetics, the concentration of one or more anesthetics is in an amount effective to mitigate pain experienced upon injection of the composition. The at least one local anesthetic can be selected from the group of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof. In one embodiment, the at least one anesthetic agent is lidocaine, such as in the form of lidocaine HCl. The compositions described herein may have a lidocaine concentration of between about 0.1% and about 5% by weight of the composition, for example, about 0.2% to about 1.0% by weight of the composition. In one embodiment, the composition has a lidocaine concentration of about 0.3% by weight (w/w %) of the composition. The concentration of lidocaine in the compositions described herein can be therapeutically effective meaning the concentration is adequate to provide a therapeutic benefit without inflicting harm to the patient.

Further, a method of preparing a HA-based composition is provided wherein the method comprises providing a precursor composition including a substantially pH neutral, crosslinked HA-based gel and adjusting the pH of the gel to a pH of greater than about 7.2, for example, about 7.5 to about 8.0. The method further comprises the step of combining a solution containing lidocaine, for example in the form of lidocaine HCl, with the slightly alkaline gel after the pH has been so adjusted and obtaining a HA-based composition including lidocaine that is stable to autoclaving.

Another method of preparing a HA-based composition containing an effective amount of lidocaine, generally comprises the steps of: providing purified NaHA material, for example, in the form of fibers; hydrating the material; and crosslinking the hydrated material with a suitable crosslinking agent to form a crosslinked HA-based gel. The method further comprises the steps of neutralizing and swelling the gel, and adding to the gel a solution containing lidocaine, preferably an acidic salt of lidocaine chlorhydrate, to form a HA/lidocaine gel. Further still, the method further comprises homogenizing the HA/lidocaine gel, and adding a small amount of an uncrosslinked HA solution. The composition is then packaged in syringes for dispensing. The syringes are then sterilized by autoclaving at an effective temperature and pressure. In accordance with the present description, the packaged and sterilized HA/lidocaine gels exhibit enhanced stability relative to HA-based compositions including lidocaine which are made using conventional methods.

The present products and compositions are considered to be sterile when exposed to temperatures of at least about 120°C to about 130°C and/or pressures of at least about 12 pounds per square inch (PSI) to about 20 PSI during autoclaving for a period of at least about 1 minute to about 15 minutes.

The present products and compositions also remain stable when stored for long periods of time at room temperature. Preferably, the present compositions remain stable for a period of at least about two months, or at least about six months, or at least about 9 months, or at least about 12 months, or at least about 24 months or at least about 36 months, at temperatures of at least about 25°C. In a specific embodiment, the compositions are stable at a temperature up to about 45°C for a period of at least two months.

The manufacturing process includes, in one embodiment, the initial step of providing raw HA material in the form of dry HA fibers or powder. The raw HA material may be HA, its salts and/or mixtures thereof. In a preferred embodiment, the HA material comprises fibers or powder of NaHA, for example, bacterial-sourced NaHA fibers. In some aspects of the present description, the HA material may be animal derived. The HA material may be a combination of raw materials including HA and at least one other polysaccharide, for example, glycosaminoglycan (GAG).

In a broad aspect of the invention, the HA material of the compositions may comprise between about 5% to about 95% low molecular weight HA with the balance of the HA material including high molecular weight HA.

In one aspect of the invention, the HA material in the compositions nearly entirely comprises or consists of low molecular weight HA. In some embodiments, nearly 100% of the HA material in the present compositions may be low molecular weight HA as defined above. In other embodiments, the HA material in the compositions comprises a combination of relatively high molecular weight HA and relatively low molecular weight HA, as defined above. In certain embodiments, at least 50%, for example, at least about 70%, for example, about 90% or greater of the HA material in the compositions is low molecular weight HA as defined above, with the remaining portion of HA being high molecular weight HA.

In one aspect of the invention, the ratio of low molecular weight to high molecular weight of the HA in the compositions is at least about 2 (w/w ≥ 2), with the low molecular weight HA having a molecular weight of between about 0.20 MDa and no greater than about 0.99 MDa.

It will be appreciated by those of ordinary skill in the art that the selection of high and low molecular weight HA material and their relative percentages or ratios is dependent upon the desired characteristics, for example, extrusion force, elastic modulus, viscous modulus and phase angle expressed as the ratio of viscous modulus to elastic modulus, cohesivity, etc. of the final HA-based product. For additional information that may be helpful in understanding this and other aspects of the present disclosure, see Lebreton, U.S. Patent Application Publication No. 2006/0194758, the entire disclosure of which is incorporated herein by this reference.

In one embodiment, the pure, dry NaHA fibers are hydrated in an alkaline solution to produce an uncrosslinked NaHA gel. Any suitable alkaline solution may be used to hydrate the NaHA in this step, for example, but not limited to aqueous solutions containing sodium hydroxide (NaOH), potassium hydroxide (KOH), sodium bicarbonate ($NaHCO_3$), lithium hydroxide (LiOH), and the like. In another embodiment, the suitable alkaline solution is aqueous solutions containing NaOH. The resulting alkaline gel will have a pH above 7.5. The pH of the resulting alkaline gel can have a pH greater than 9, or a pH greater than 10, or a pH greater than 12, or a pH greater than 13.

The next step in the manufacturing process involves the step of crosslinking the hydrated, alkaline NaHA gel with a suitable crosslinking agent. The crosslinking agent may be any agent known to be suitable for crosslinking polysaccharides and their derivatives via their hydroxyl groups. Suitable crosslinking agents include but are not limited to, 1,4-butanediol diglycidyl ether (or 1,4-bis(2,3-epoxypropoxy)butane or 1,4-bisglycidyloxybutane, all of which are commonly known as BDDE), 1,2-bis(2,3-epoxypropoxy)ethylene and 1-(2,3-epoxypropyl)-2,3-epoxycyclohexane. The use of more than one crosslinking agent or a different crosslinking agent is not excluded from the scope of the present disclosure. In one aspect of the present disclosure, the HA gels described herein are crosslinked using BDDE.

The step of crosslinking may be carried out using any means known to those of ordinary skill in the art. Those skilled in the art appreciate how to optimize conditions of crosslinking according to the nature of the HA, and how to carry out crosslinking to an optimized degree.

In another embodiment, the crosslinking of the HA is accomplished during hydration of the HA fibers, by hydrating the combined high and low molecular weight fibers in an alkaline solution containing a crosslinking agent, for example, BDDE.

The degree of crosslinking in the HA component of the present compositions is at least about 1% and is up to about 20% BDDE/HA, w/w, for example, between about 4% and about 12%, for example, about 10 %, for example, about 8%, for example, about 6%, for example, about 5%, for example, about 4%.

The hydrated crosslinked, HA gels may be swollen to obtain the desired HA concentration. This step can be accomplished by neutralizing the crosslinked, hydrated HA gel, for example by adding an aqueous solution containing of an acid, such as HCl. The gels are then swelled in a phosphate buffered saline (PBS) solution for a sufficient time and at a low temperature.

The gels may now be purified by conventional means such as, dialysis against a phosphate buffer, or alcohol precipitation, to recover the crosslinked material, to stabilize the pH of the material and to remove any un-reacted crosslinking agent. Additional water or a slightly alkaline aqueous solution can be added to bring the concentration of the HA in the composition to a desired concentration. In some embodiments, the HA concentration of the crosslinked component of the compositions is adjusted to between about 15 mg/g and about 20 mg/g. For example, the HA concentration of the crosslinked portion of the compositions may be adjusted to yield an HA concentration of about 15 mg/g, about 17 mg/g or about 20 mg/g.

The pH of the purified, substantially pH neutral, crosslinked HA gels are preferably adjusted to cause the gel to become slightly alkaline such that the gels have a pH of greater than about 7.2, for example, about 7.5 to about 8.0. This step may be accomplished by any suitable means, for example, by adding a suitable amount of dilute NaOH, KOH, NaHCO$_3$ or LiOH, to the gels or any other alkaline molecule, solution and/or buffering composition know by one skilled in the art.

In some embodiments, an effective amount of a local anesthetic, for example, lidocaine, such as lidocaine HCl, is then added to the purified NaHA gels. For example, in some embodiments, the lidocaine HCl is provided in a powder form which is solubilized using water for injection (WFI). The gels are kept neutral with a buffer or by adjustment with diluted NaOH in order that the final HA/lidocaine composition will have a desired, substantially neutral pH. Preferably, the final HA-based filler compositions including lidocaine will have a lidocaine concentration of between at least about 0.1% and about 5%, for example, about 2% by weight of the composition, or in another example about 0.3%.

After the addition of the lidocaine HCl, or alternatively, during the addition of the lidocaine HCl, the HA/lidocaine gels, or compositions, are homogenized to create highly homogenous HA/lidocaine gels having a desired consistency and stability. Preferably, the homogenization step comprises mixing, stirring, or beating the gels with a controlled shearing force obtaining substantially homogenous mixtures.

The HA/lidocaine compositions described herein display a viscosity which is dependent on the composition's properties and the presence of at least one anesthetic agent. The viscosity of the HA/lidocaine composition can be from about 50 Pa*s to about 450 Pa*s. In other embodiments, the viscosity can be from about 100 Pa*s to about 400 Pa*s, or about from 150 Pa*s to about 350 Pa*s.

In one embodiment, after homogenizing the HA composition, an amount of uncrosslinked HA solution or gel is added to the composition.

In this aspect of the invention, the added uncrosslinked HA solution has a HA concentration of between about 10 mg/g and about 50 mg/g, for example, about 10 mg/g, about 20 mg/g, about 30 mg/g, about 40 mg/g, about 50 mg/g. The HA in the uncrosslinked solution is a high molecular weight HA, having a molecular weight of at least about 1.0 MDa up to about 4.0 MDa, for example, a molecular weight of about 1.2 MDa, about 1.4 MDa, about 1.6 MDa, about 1.8 MDa, about 2.0 MDa, about 2.2 MDa about 2.4 MDa, about 2.6 MDa, about 2.8 MDa, about 3.0 MDa, about 3.2 MDa, about 3.4 MDa, about 3.6 MDa, or about 3.8. The uncrosslinked HA solution is added to the crosslinked HA component, to produce a dermal filler composition having a final weight percent of between 0.5% and 10% uncrosslinked HA.

For example, HA fibers having a molecular weight of about 2.0 MDa are swollen in a phosphate buffer solution to obtain an uncrosslinked HA gel having a HA concentration of about 20 mg/g. This uncrosslinked HA gel is then is added to the crosslinked HA gel composition during a final mixing step to obtain a dermal filler composition having an uncrosslinked HA gel of about 1.0% w/w.

After adding the uncrosslinked HA gel to the crosslinked HA gel, the compositions are introduced into syringes and sterilized. Syringes useful according to the present description include any syringe known in the art capable of delivering viscous dermafiller compositions. The syringes generally have an internal volume of about 0.4 mL to about 3 mL, more preferably between about 0.5 mL and about 1.5 mL or between about 0.8 mL and about 2.5 mL. This internal volume is associated with an internal diameter of the syringe which plays a key role in the extrusion force needed to inject high viscosity dermal filler compositions. The internal diameters are generally about 4 mm to about 9 mm, more preferably from about 4.5 mm to about 6.5 mm or from about 4.5 mm to about 8.8 mm. Further, the extrusion force needed to deliver the HA compositions from the syringe is dependent on the needle gauge. The gauges of needles used generally include gauges between about 18G and about 40G, more preferably about 25G to about 33G, or from about 25G to about 30G. A person of ordinary skill in the art can determine the correct syringe dimensions and needle gauge required to arrive at a particular extrusion force requirement.

The extrusion forces displayed by the HA compositions described herein using the needle dimensions described above are at an injection speeds that are comfortable to a patient. Comfortable to a patient is used to define a rate of injection that does not injure or cause excess pain to a patient upon injection to the soft tissue. One skilled in the art will appreciate that comfortable as used herein includes not only patient comfort, but also comfort and ability of the physician or medical technician injecting the HA compositions. Although certain extrusion forces may be achievable with the HA compositions of the present description, one skilled in the art understands that high extrusion forces can lead to lack of control during injection and that such lack of control may result in additional pain to the patient. Extrusion forces of the present HA/lidocaine compositions can be from about 5 N to about 20 N, or more preferably from about 8 N to about 15 N, when extruded at 13.5 mm/min in a 1.0 mL syringe with a standard needle of 30G$^{1/2}$.

Sterilization, as used herein comprises any method known in the art to effectively kill or eliminate transmissible agents, preferably without substantially altering of degrading the HA/lidocaine compositions.

One preferable method of sterilization of the filled syringes is by autoclave. Autoclaving can be accomplished by applying a mixture of heat, pressure and moisture to a sample in need of sterilization. Many different sterilization temperatures, pressures and cycle times can be used for this step. For example, the filled syringes may be sterilized at a temperature of at least about 120°C to about 130°C or greater. Moisture may or may not be utilized. The pressure applied is in some embodiments depending on the temperature used in the sterilization process. The sterilization cycle may be at least about 1 minute to about 20 minutes or more.

Another method of sterilization incorporates the use of a gaseous species which is known to kill or eliminate transmissible agents. Preferably, ethylene oxide is used as the sterilization gas and is known in the art to be useful in sterilizing medical devices and products.

A further method of sterilization incorporates the use of an irradiation source which is known in the art to kill or eliminate transmissible agents. A beam of irradiation is targeted at the syringe containing the HA composition, and the wavelength of energy kills or eliminates the unwanted transmissible agents. Preferable energy useful include, but is not limited to ultraviolet (UV) light, gamma irradiation, visible light, microwaves, or any other wavelength or band of wavelengths which kills or eliminates the unwanted transmissible agents, preferably without substantially altering of degrading the HA composition.

**EXAMPLE 1**

**Manufacture of a Low Molecular Weight Soft Filler of the Invention including Lidocaine**

90% of NaHA fibers or powder having a low molecular weight and 10% of NaHA fibers or powder having a high molecular weight, (ratio of high molecular weight to low molecular weight of 2:1) are hydrated in an alkaline solution, for example, an aqueous solution containing NaOH. The mixture is mixed at ambient temperature, about 23°C, to form a substantially homogenous, alkaline HA gel.

A crosslinking agent, BDDE, is diluted in an aqueous solution and added to the alkaline HA gel. The mixture is homogenized for several minutes.

The resulting crosslinked HA gel mixture is then heated at about 50°C for about 3 hours. The material is now a highly crosslinked HA/BDDE gel (aspect = solid gel). This crosslinked gel is then neutralized with a suitable acidic solution. The neutralized HA gel is then swollen in a phosphate buffer at a cold temperature, for example a temperature of about 5°C, to obtain a highly cohesive HA gel. In this specific example, the phosphate buffered saline solution contains water-for-injection (WFI), disodium hydrogen phosphate, and sodium dihydrogen phosphate. When neutralized and swollen, the water absorbed by the crosslinked HA component is in a weight ratio of at least 1:1, and without the gel breaking into pieces.

The swollen HA gel is then mechanical stirred and filled into dialysis membranes and dialyzed against a phosphate buffer. The HA gel is filled into dialysis membranes and dialyzed against a phosphate buffer for up to several days with regular changes of the bath, in order to remove the un-reacted crosslinker, to stabilize the pH close to neutrality (pH=7.2) and to ensure proper osmolarity of the HA gel. The osmolarity of the resulting HA gel is between about 200 mOsmol and about 400 mOsmol, most preferably about 300 mOsmol.

After dialysis, the resulting HA gel has a substantially neutral pH, preferably about 7.2.

Lidocaine chlorhydrate (lidocaine HCl) in powder form is first solubilized in WFI and filtered through a 0.2 $\mu$m filter. Dilute NaOH solution is added to the HA gel in order to reach a slightly basic pH (for example, a pH of between about 7.5 and about 8). The lidocaine HCl solution is then added to the slightly basic gel to reach a final desired concentration, for example, a concentration of about 0.3% (w/w). The resulting pH of the HA/lidocaine mixture is then about 7 and the HA concentration is about 24 mg/mL. Mechanical mixing is performed in order to obtain a proper homogeneity in a standard reactor equipped with an appropriate blender mechanism.

An amount of uncrosslinked HA gel is added to the HA/lidocaine gel mixture. Specifically, high molecular weight HA fibers are swollen in a phosphate buffer solution, in order to obtain a homogeneous viscoelastic gel. This uncrosslinked HA gel is then added to the crosslinked HA/lidocaine gel (for example, at about 1%, w/w). The resulting gel is then filled into Ready-to-Fill sterile syringes and autoclaved at sufficient temperatures and pressures for sterilization for at least about 1 minute.

After autoclaving, the final HA/lidocaine product is packaged and distributed to physicians. The autoclaved HA/lidocaine product has a viscosity, cohesivity, and extrusion force that are acceptable. No degradation of the HA/lidocaine gel product is found during testing of the product after the product has spent several months in storage.

**EXAMPLE 2**

**Manufacture of a Low Molecular Weight Soft Filler of the Invention (without Lidocaine)**

0.8 g of predried fibers of sodium hyaluronate (NaHA) of having a molecular weight of about 0.2 MDa is weighed out into a first receptacle.

0.2 g of predried fibers of NaHA, of having a molecular weight of about 2.4 MDa is weighed out into another receptacle.

The two different grades of NaHA are combined.

In a separate receptacle the chosen crosslinking agent, 1,4-butanediol diglycidyl ether (BDDE), is diluted in 1% sodium hydroxide solution.

6.8 g of the previously prepared BDDE solution diluted to 1/100 are then added to the mixed NaHA fibers, still in the solid state, and the mixture is homogenized mechanically with a spatula. The mixture is mixed for two hours at about 20°C to for a substantially homogenous, alkaline HA gel.

The mixture is then placed in a warm water bath at 50°C for 2 to 3 hours, with further homogenization after 15 minutes of immersion.

The resulting crosslinked HA polymer is then immersed in a phosphate buffer (PB) to stabilize the pH.

The swollen crosslinked NaHA polymer is then purified by immersion in different baths of phosphate buffer to remove unreacted crosslinking agent and HA.

Dry HA material having a high molecular weight is hydrated in 1 liter of water to obtain an uncrosslinked HA gel. 1% of this HA gel corresponding to the amount in the final composition is mixed into the crosslinked HA gel to provide a dermal filler composition in accordance with the present invention.

The hydrogel obtained is then homogenized mechanically to ensure the final homogeneity, and packed into syringes which are sterilized in an autoclave.

The gel obtained is a long lasting injectable composition using a fine gauge needle (e.g. 30 Gauge or 33 Gauge) to improve nasolabial fold lines on the face.

Although the invention has been described and illustrated with a certain degree of particularity, it is understood that the present disclosure has been made only by way of example, and that numerous changes in the combination and arrangement of parts can be resorted to by those skilled in the art without departing from the scope of the invention, as hereinafter claimed.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

Embodiments

1. A soft tissue filler composition comprising:

(1) a crosslinked hyaluronic acid (HA) component comprising a crosslinked mixture of (i) a first, low molecular weight HA material having a weight average molecular weight of between 0.20 about MDa and about 0.99 MDa and (ii) a second high molecular weight HA material having a weight average molecular weight of between about 1.0 MDa and about 4.0 MDa;

wherein the weight average molecular weight of the second, high molecular weight HA material is at least twice that of the first, low molecular weight HA material;
wherein the HA materials are hyaluronic acid or one or more salts thereof;

wherein the crosslinked HA component has a HA concentration of between about 15.0 mg/g and about 20.0 mg/g; and

wherein the crosslinking is achieved by use of a crosslinking agent; and

(2) an uncrosslinked HA component which is HA or a salt thereof, and which has a weight average molecular weight of at least about 1.0 MDa and ispresent in the composition in an amount of less than about 5.0% w/w

2. The composition of 1 wherein the uncrosslinked HA component has a weight average molecular weight of at least about 2.0 MDa.

3. The composition of 1 wherein the weight average molecular weight of the first, low molecular weight HA material is at least about 500,000 Da.

4. The composition of 1 wherein the weight average molecular weight of the second, high molecular weight HA material is at least about 1.0M Da.

5. The composition of 1 wherein the weight average molecular weight of the first, low molecular weight HA material is about 800,000 Da.

6. The composition of 1 wherein the mixture contains about 50% by weight of the first, low molecular weight HA material.

7. The composition of 1 wherein the mixture contains at least about 70% by weight of the first, low molecular weight HA material.

8. The composition of 1 wherein the mixture contains about 90% by weight of the first, low molecular weight HA material.

9. The composition of 1 wherein the crosslinking agent is selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether.

10. The composition of 1 wherein the crosslinked HA component has a HA concentration of about 15.0 mg/g.

11. The composition of 1 wherein the crosslinked HA component has a HA concentration of about 17.5 mg/g.

12. The composition of 1 wherein the uncrosslinked HA component is present in an amount of less than about 1.0 % w/w.

13. The composition of 1 wherein the uncrosslinked HA component has a HA concentration of between about 10.0 mg/g and about 50.0 mg/g.

14. The composition of 1 further comprising at least one anesthetic agent combined with said crosslinked HA component.

15. A soft tissue filler composition comprising:

a crosslinked hyaluronic acid (HA) component comprising a mixture of a first, low molecular weight HA material and a second, high molecular weight HA material, the materials being hyaluronic acid or salts thereof and being crosslinked with a crosslinking agent, the crosslinked HA component having a HA concentration of between about 15 mg/g and about 20 mg/g, and the mixture containing at least 50% by weight of the low molecular weight HA material;
the weight average molecular weight of the second, high molecular weight HA material being at least twice that of the weight average molecular weight of the first, low molecular weight HA material;
an uncrosslinked hyaluronic acid component or salt thereof, having a weight average molecular weight of at least about 2.4 MDa and an HA concentration of between about 10 mg/g and about 50 mg/g;
the uncrosslinked HA component being present in an amount of less than about 5.0 % w/w; and
at least one anesthetic agent combined with said crosslinked HA component.

16. The composition of 15 wherein the mixture contains at least 70% by weight of the first, low molecular weight HA material.

17. The composition of 15 wherein the mixture contains about 90% by weight of the first, low molecular weight HA material.

18. A method of making a soft tissue filler composition comprising the steps of:

preparing a crosslinked hyaluronic acid (HA) gel comprising a mixture of a first, low molecular weight HA material and a second, high molecular weight HA material, the second, high molecular weight HA material having a weight average molecular weight of at least twice that of the first, low molecular weight material, the mixture comprising more than about 50% by weight of the first, low molecular weight HA material, wherein the HA materials are hyaluronic acid or one or more salts thereof;
adjusting the HA concentration in the crosslinked HA gel to a HA concentration of between about 15.0 mg/g and about 20.0 mg/g;
preparing a solution comprising an uncrosslinked HA material or a salt thereof having a weight average molecular weight of between at least about 1.0 MDa and about 4.0 MDa and a concentration of between about 10 mg/g and about 50 mg/g; and
combining the gel with the solution.

19. The method of 18 wherein the mixture comprises more than about 70% by weight of the first, low molecular weight HA material.

20. The method of 18 wherein the mixture comprises more than about 90% by weight of the first, low molecular weight HA material.

21. The method of 18 further comprising the step of combining at least one anesthetic agent with the gel.

22. The method of 21 wherein the step of combining at least one anesthetic agent with the gel is performed prior to the step of combining the gel with the solution.

**Claims**

1.  A soft tissue filler composition comprising:

(1) a crosslinked hyaluronic acid (HA) component comprising a crosslinked mixture of (i) a first, low molecular weight HA material having a weight average molecular weight of between 0.20 MDa and 0.99 MDa, and (ii) a second, high molecular weight HA material having a weight average molecular weight of between 1.0 MDa and 4.0 MDa;

wherein the weight average molecular weight of the second, high molecular weight HA material is at least twice that of the first, low molecular weight HA material;
wherein the HA materials are hyaluronic acid or one or more salts thereof;
wherein the crosslinked HA component has a HA concentration of between 15.0 mg/g and 20.0 mg/g; and
wherein the crosslinking is achieved by use of a crosslinking agent; and

(2) an uncrosslinked HA component which is HA or a salt thereof, and which has a weight average molecular weight of at least 2.0 MDa and is present in the composition in an amount of less than 5.0% w/w.

2.  The composition of claim 1, wherein the weight average molecular weight of the first, low molecular weight HA material is between 0.3 MDa and 0.75 MDa.

3.  The composition of claim 1, wherein the weight average molecular weight of the second, high molecular weight HA material is in the range of 1.5 MDa to 3.0 MDa.

4.  The composition of claim 1, wherein the mixture contains more than 50% by weight of the first, low molecular weight HA material.

5. The composition of claim 1, wherein the mixture contains at least 70% by weight of the first, low molecular weight HA material.

6. The composition of claim 1, wherein the mixture contains about 90% by weight of the first, low molecular weight HA material.

7. The composition of claim 1, wherein the crosslinking agent is selected from 1,4-butanediol diglycidyl ether (BDDE), 1,4-bis(2,3-epoxypropoxy)butane, 1,4-bisglycidyloxybutane, 1,2-bis(2,3-epoxypropoxy)ethylene, 1-(2,3-epoxypropyl)-2,3-epoxycyclohexane, and 1,4-butanediol diglycidyl ether.

8. The composition of claim 1, wherein the uncrosslinked HA component is present in an amount of less than 2.0% w/w.

9. The composition of claim 1, wherein the uncrosslinked HA component is present in an amount of less than 1.0% w/w.

10. The composition of claim 1, wherein the uncrosslinked HA component has a HA concentration of between 10.0 mg/g and 50.0 mg/g.

11. The composition of claim 1, further comprising at least one anesthetic agent combined with said crosslinked HA component.

Europäisches
Patentamt

European
Patent Office

Office européen
des brevets

# EUROPEAN SEARCH REPORT

**Application Number**

**EP 22 19 9080**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/255068 A1 (STROUMPOULIS DIMITRIOS [US] ET AL) 7 October 2010 (2010-10-07) * page 2, paragraph 20-21 * * page 2, paragraph 28 – page 3, paragraph 43 * * example 3 * * claims * | 1-11 | INV. A61L27/26 A61L27/50 A61L27/52 |
| A | US 2010/028437 A1 (LEBRETON PIERRE F [FR]) 4 February 2010 (2010-02-04) * page 3, paragraph 41-43 * * page 4, paragraph 48-52 * * page 4, paragraph 60 – page 5, paragraph 62 * * claims * | 1-11 | |
| A | US 2006/194758 A1 (LEBRETON PIERRE [FR]) 31 August 2006 (2006-08-31) * claims * | 1-11 | |
| A | WO 2008/068297 A1 (FABRE PIERRE DERMO COSMETIQUE [FR]; PIRON ESTELLE [FR]) 12 June 2008 (2008-06-12) * claims * | 1-11 | TECHNICAL FIELDS SEARCHED (IPC) A61L A61K A61Q |

The present search report has been drawn up for all claims

| Place of search | Date of completion of the search | Examiner |
|---|---|---|
| The Hague | 22 February 2023 | Van den Bulcke, H |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 19 9080

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | | Publication date | Patent family member(s) | | | Publication date |
|---|---|---|---|---|---|---|
| US 2010255068 | A1 | 07-10-2010 | AU | 2010232547 | A1 | 27-10-2011 |
| | | | CA | 2757557 | A1 | 07-10-2010 |
| | | | DK | 2413894 | T3 | 03-04-2017 |
| | | | EP | 2413894 | A2 | 08-02-2012 |
| | | | ES | 2621380 | T3 | 03-07-2017 |
| | | | US | 2010255068 | A1 | 07-10-2010 |
| | | | US | 2012034462 | A1 | 09-02-2012 |
| | | | US | 2014094431 | A1 | 03-04-2014 |
| | | | WO | 2010115081 | A2 | 07-10-2010 |
| US 2010028437 | A1 | 04-02-2010 | AU | 2009278883 | A1 | 11-02-2010 |
| | | | AU | 2009278884 | A1 | 11-02-2010 |
| | | | BR | PI0917573 | B1 | 13-03-2018 |
| | | | BR | PI0917588 | B1 | 13-03-2018 |
| | | | CA | 2732788 | A1 | 11-02-2010 |
| | | | CA | 2732928 | A1 | 11-02-2010 |
| | | | CA | 2944734 | A1 | 11-02-2010 |
| | | | CA | 3023168 | A1 | 11-02-2010 |
| | | | CA | 3112106 | A1 | 11-02-2010 |
| | | | CN | 102170855 | A | 31-08-2011 |
| | | | CN | 102170856 | A | 31-08-2011 |
| | | | CN | 103285423 | A | 11-09-2013 |
| | | | CY | 1118281 | T1 | 28-06-2017 |
| | | | CY | 1118821 | T1 | 10-01-2018 |
| | | | DK | 2323617 | T3 | 24-04-2017 |
| | | | DK | 2326302 | T3 | 14-11-2016 |
| | | | DK | 2674147 | T3 | 30-03-2020 |
| | | | EP | 2323617 | A1 | 25-05-2011 |
| | | | EP | 2326302 | A1 | 01-06-2011 |
| | | | EP | 2674147 | A1 | 18-12-2013 |
| | | | EP | 3205332 | A1 | 16-08-2017 |
| | | | EP | 3662889 | A1 | 10-06-2020 |
| | | | EP | 3988082 | A1 | 27-04-2022 |
| | | | ES | 2599763 | T3 | 03-02-2017 |
| | | | ES | 2622708 | T3 | 07-07-2017 |
| | | | ES | 2780190 | T3 | 24-08-2020 |
| | | | HK | 1156883 | A1 | 22-06-2012 |
| | | | HK | 1189518 | A1 | 13-06-2014 |
| | | | HU | E031483 | T2 | 28-07-2017 |
| | | | HU | E031598 | T2 | 28-07-2017 |
| | | | HU | E048366 | T2 | 28-08-2020 |
| | | | HU | E053942 | T2 | 30-08-2021 |
| | | | JP | 5670899 | B2 | 18-02-2015 |
| | | | JP | 5670900 | B2 | 18-02-2015 |
| | | | JP | 5808848 | B2 | 10-11-2015 |
| | | | JP | 2011529762 | A | 15-12-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 19 9080

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | Publication date | Patent family member(s) | Publication date |
|---|---|---|---|
| | | JP 2011529763 A | 15-12-2011 |
| | | JP 2014237718 A | 18-12-2014 |
| | | JP 2014237719 A | 18-12-2014 |
| | | JP 2016000350 A | 07-01-2016 |
| | | KR 20110040966 A | 20-04-2011 |
| | | KR 20110043730 A | 27-04-2011 |
| | | MX 342613 B | 06-10-2016 |
| | | PL 2323617 T3 | 31-08-2017 |
| | | PL 2326302 T3 | 31-01-2017 |
| | | PL 2674147 T3 | 16-11-2020 |
| | | PT 2323617 T | 24-04-2017 |
| | | PT 2326302 T | 02-11-2016 |
| | | RU 2011107877 A | 10-09-2012 |
| | | RU 2011107878 A | 10-09-2012 |
| | | SG 10201505441T A | 28-08-2015 |
| | | SG 10202104803S A | 29-06-2021 |
| | | US 2010028437 A1 | 04-02-2010 |
| | | US 2010028438 A1 | 04-02-2010 |
| | | US 2011118206 A1 | 19-05-2011 |
| | | US 2012172328 A1 | 05-07-2012 |
| | | US 2013041038 A1 | 14-02-2013 |
| | | US 2013041039 A1 | 14-02-2013 |
| | | US 2013102563 A1 | 25-04-2013 |
| | | US 2013131011 A1 | 23-05-2013 |
| | | US 2013244970 A1 | 19-09-2013 |
| | | US 2014148406 A1 | 29-05-2014 |
| | | US 2014213546 A1 | 31-07-2014 |
| | | US 2014213547 A1 | 31-07-2014 |
| | | US 2015045321 A1 | 12-02-2015 |
| | | US 2015297790 A1 | 22-10-2015 |
| | | US 2016279296 A1 | 29-09-2016 |
| | | US 2018000992 A1 | 04-01-2018 |
| | | US 2019076579 A1 | 14-03-2019 |
| | | US 2019076580 A1 | 14-03-2019 |
| | | US 2019343990 A1 | 14-11-2019 |
| | | US 2019350832 A1 | 21-11-2019 |
| | | US 2020086009 A1 | 19-03-2020 |
| | | US 2021283313 A1 | 16-09-2021 |
| | | US 2022313870 A1 | 06-10-2022 |
| | | WO 2010015900 A1 | 11-02-2010 |
| | | WO 2010015901 A1 | 11-02-2010 |
| US 2006194758 A1 | 31-08-2006 | BR PI0409762 A | 09-05-2006 |
| | | CA 2521961 A1 | 28-10-2004 |
| | | EP 1611160 A2 | 04-01-2006 |
| | | EP 2289945 A1 | 02-03-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 19 9080

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | Publication date | Patent family member(s) | | Publication date |
|---|---|---|---|---|
| | | EP 2868669 A1 | | 06-05-2015 |
| | | ES 2536564 T3 | | 26-05-2015 |
| | | FR 2861734 A1 | | 06-05-2005 |
| | | JP 4827727 B2 | | 30-11-2011 |
| | | JP 2006522851 A | | 05-10-2006 |
| | | US 2006194758 A1 | | 31-08-2006 |
| | | US 2010226988 A1 | | 09-09-2010 |
| | | US 2012295870 A1 | | 22-11-2012 |
| | | US 2014011990 A1 | | 09-01-2014 |
| | | US 2015065701 A1 | | 05-03-2015 |
| | | US 2016317571 A1 | | 03-11-2016 |
| | | US 2019022130 A1 | | 24-01-2019 |
| | | US 2020246375 A1 | | 06-08-2020 |
| | | US 2021322460 A1 | | 21-10-2021 |
| | | WO 2004092222 A2 | | 28-10-2004 |
| WO 2008068297 A1 | 12-06-2008 | AR 064175 A1 | | 18-03-2009 |
| | | AU 2007328917 A1 | | 12-06-2008 |
| | | BR PI0720176 A2 | | 24-12-2013 |
| | | CA 2671793 A1 | | 12-06-2008 |
| | | CN 101594892 A | | 02-12-2009 |
| | | EP 2152329 A1 | | 17-02-2010 |
| | | EP 2489374 A1 | | 22-08-2012 |
| | | ES 2632947 T3 | | 18-09-2017 |
| | | FR 2909560 A1 | | 13-06-2008 |
| | | IL 199002 A | | 31-01-2013 |
| | | JP 5642388 B2 | | 17-12-2014 |
| | | JP 2010511454 A | | 15-04-2010 |
| | | KR 20090085102 A | | 06-08-2009 |
| | | KR 20140101018 A | | 18-08-2014 |
| | | MA 30978 B1 | | 01-12-2009 |
| | | NZ 577917 A | | 12-01-2012 |
| | | RU 2009125201 A | | 20-01-2011 |
| | | TN 2009000223 A1 | | 18-10-2010 |
| | | TW 200836778 A | | 16-09-2008 |
| | | UA 95660 C2 | | 25-08-2011 |
| | | US 2010316683 A1 | | 16-12-2010 |
| | | WO 2008068297 A1 | | 12-06-2008 |
| | | ZA 200903919 B | | 30-06-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060194758, Lebreton